# FASCICULE DE BREVET EUROPEEN

(11) **EP 3 228 339 B1**
(45) Date de publication et mention de la délivrance du brevet: **04.12.2019**
(21) Numéro de dépôt: 17000603.5
(22) Date de dépôt: 10.04.2017
(51) Int. Cl.: A61M 1/16, G01N 1/20

(54) **DISPOSITIF DE RECUEIL D'UN ECHANTILLON DE DIALYSAT**
VORRICHTUNG ZUR PROBENSAMMLUNG EINES DIALYSATS
DEVICE FOR COLLECTING A DIALYSATE SAMPLE

(30) Priorité: 08.04.2016 FR 1653119
(43) Date de publication de la demande: 11.10.2017
(73) Titulaire: THERADIAL societe par actions simplifiees, 44700 Orvault (FR)
(72) Inventeur: Duval, Bruno, 44000 Nantes (FR); Le Pottier, Frédéric, 44700 Orvault (FR)
(74) Mandataire: Corret, Hélène

(56) Documents cités:
- EP-A1- 0 069 029
- WO-A1-93/02346
- FR-A1- 2 696 644
- AVIRAM A ET AL: "DIALYSANCE OF AMINO ACIDS AND RELATED SUBSTANCES", NEPHRON, S. KARGER AG, SWITZERLAND, vol. 8, 1 janvier 1971 (1971-01-01), pages 440-454, XP000564952, ISSN: 0028-2766, DOI: 10.1159/000179948

## Description

La présente invention concerne le domaine de l'hémodialyse et plus particulièrement un dispositif d'échantillonnage du liquide de dialyse en sortie d'un rein artificiel ou hémodialyseur.

On sait que les patients souffrant d'insuffisance rénale chronique sont soumis périodiquement à une séance d'hémodialyse. Celle-ci consiste à épurer le sang du patient en le faisant circuler, de façon extracorporelle, au contact d'une face d'une membrane semi-perméable dont l'autre face est parcourue par un liquide de traitement sensiblement isotonique au sang.

En pratique, un hémodialyseur se présente généralement sous la forme d'un cylindre dans lequel sont disposées des fibres creuses dont le diamètre est inférieur à 100 µm. Le sang du patient circule ainsi au travers de ces fibres et la circulation du liquide de traitement s'effectue en sens inverse pour optimiser les échanges.

Il s'établit ainsi un transfert diffusif au travers de la membrane pour les substances ayant des concentrations différentes de part et d'autre de la membrane. C'est ainsi que les impuretés du sang telles que l'urée, migrent du sang vers le liquide de traitement.

Il est nécessaire d'évaluer l'efficacité d'une séance de dialyse pour l'adapter à chaque patient. A cet effet, il convient de mesurer la quantité d'impuretés extraite du sang, au cours de la séance de dialyse.

Par ailleurs, les fabricants de dialyseurs communiquent leurs performances, en indiquant des degrés de filtration minimum de certaines substances comme l'urée ou l'albumine.

Cependant, il n'existe pas à ce jour de dispositif permettant d'évaluer objectivement l'efficacité de ces hémodialyseurs.

Les performances des hémodialyseurs ne sont par ailleurs valables que pour des conditions précises d'utilisation qui ne sont pas nécessairement reproduites lors d'une séance de dialyse.

Il n'est donc pas possible de connaitre de façon précise la quantité de substances épurées par dialyse, en procédant par calcul à partir des performances nominales de l'hémodialyseur.

Il a donc été envisagé de recueillir en totalité le liquide de dialyse usé, d'en extraire un échantillon, puis d'analyser cet échantillon pour déterminer la teneur en une substance particulière. La quantité totale de substance ainsi épurée peut alors être facilement obtenue.

Cette solution pose de nombreux problèmes pratiques dans la mesure où le volume de liquide usé produit au cours d'une séance de dialyse est de l'ordre de 120 litres.

C'est pourquoi, il a été proposé de nombreux dispositifs permettant l'analyse du liquide usé sans nécessiter le recueil de la totalité de ce liquide.

Ainsi, le document EP 1 083 938 décrit un dispositif permettant de mesurer en continu la concentration d'une substance déterminée dans le liquide de dialyse usé.

Ce dispositif présente l'inconvénient de ne pas permettre de recueillir un échantillon représentatif de la totalité du liquide usé évacué lors d'une opération de dialyse.

D'autres dispositifs ont été proposés pour recueillir un échantillon de liquide usé, représentatif de la totalité du liquide évacué en sortie d'un rein artificiel, au cours d'une séance de dialyse.

On peut citer le document FR 2 739 780. Il concerne un dispositif pour recueillir un échantillon de liquide de dialyse usé qui comprend un moyen de pompage disposé sur une canalisation principale reliée, en entrée, à la sortie du rein artificiel, une canalisation de prélèvement étant reliée à cette canalisation principale, en sortie de la pompe. La canalisation de prélèvement débouche sur un dispositif de recueil de l'échantillon, tandis que la canalisation principale est reliée à l'égout.

La répartition des flux entre la canalisation principale et la canalisation de prélèvement est réalisée grâce aux informations données par deux compteurs volumétriques situés chacun sur une de ces canalisations.

Ce dispositif présente l'inconvénient principal de ne pouvoir fonctionner que lors d'un fonctionnement régulier du rein artificiel. En effet, lorsque le débit de liquide usé comporte des irrégularités ou lorsque des dégazages se produisent, la pompe ne peut pas fonctionner normalement et c'est le fonctionnement de l'ensemble du dispositif qui est perturbé.

Par ailleurs, ce fonctionnement nécessite la présence de deux compteurs volumétriques dans le dispositif, ce qui rend à la fois sa fabrication et son fonctionnement plus complexes. De plus, les risques de dérive entre les deux compteurs volumétriques peuvent conduire à diminuer la précision dans la constitution de l'échantillon de liquide de dialyse usé.

On peut encore citer le document FR 2 696 644 qui décrit un tel dispositif comportant un récipient de collecte du liquide de dialyse à échantillonner qui est relié à la sortie du dialyseur. C'est donc l'intégralité de ce liquide usagé qui transite par le récipient.

Le liquide présent dans le récipient est évacué en générant deux flux de débits différents grâce à des moyens de pompage, le flux de moindre débit étant destiné à être recueilli en totalité à des fins de dosage et le flux de plus grand débit étant destiné à être conduit à l'égout.

Ce dispositif est relativement complexe, notamment du fait de la présence de deux moyens de pompage différents comprenant deux soufflets actionnés par un moteur. Par ailleurs, il suppose un réglage approprié du rapport de débit entre les deux moyens de pompage, rapport qu'il n'est pas facile de maintenir de façon durable au cours de l'utilisation du dispositif, d'autant plus que ces moyens de pompage sont fragiles. Le volume total de liquide de dialyse usagé est calculé en fonction du volume de l'échantillon recueilli et du rapport de débit entre les deux flux, ce qui ne permet pas de réaliser ce calcul avec une précision suffisante.

Enfin, les opérations de désinfection du dispositif, nécessaires pour assurer une bonne représentativité de l'échantillon recueilli, sont longues du fait de la présence du récipient de collecte et des nombreuses canalisations.

On peut également citer le document FR 2 679 334 qui décrit un dispositif de recueil d'un échantillon de dialysat en vue de son analyse qui comporte un réservoir tampon traversé par la totalité du dialysat, ce réservoir étant associé à un dispositif séparateur, agencé pour prélever la proportion de dialysat à analyser et pour évacuer le restant.

Dans ce dispositif, le séparateur est en communication avec le réservoir tampon et présente des orifices calibrés de façon à répartir le liquide de dialyse usé en deux flux différents, l'un étant recueilli en vue d'une analyse et l'autre étant lui directement évacué.

Ce séparateur mécanique est conçu pour assurer la précision des mesures dans le temps mais il est relativement complexe, à la fois dans sa fabrication et dans son entretien.

Cet entretien est d'autant plus indispensable que la précision de l'échantillon est basée sur un fonctionnement identique des orifices calibrés, lesquels peuvent facilement se boucher.

Il est par ailleurs encombrant puisqu'il doit nécessairement être situé sous le réservoir tampon pour recevoir, par gravité, le liquide présent dans le réservoir tampon.

L'invention a pour objet de pallier les inconvénients de ces dispositifs en proposant un dispositif de recueil d'un échantillon représentatif du liquide de dialyse en sortie d'un rein artificiel qui soit d'une réalisation simplifiée, dont le fonctionnement soit assuré quel que soit le mode de fonctionnement du rein artificiel, sur lequel il n'a pas d'impact, ce dispositif donnant des mesures du volume de l'échantillon et du volume total du liquide usé, avec une grande précision et simplifiant considérablement les opérations de désinfection.

L'invention est définie dans les revendications. Ainsi, le dispositif selon l'invention comporte :
- un moyen de recueil et de stockage du liquide de dialyse usé, destiné à être relié par une canalisation à la sortie du dialyseur et,
- des moyens de pompage pour pomper du liquide stocké dans le moyen de recueil et de stockage,
- des moyens de répartition du liquide en deux flux différents, l'un destiné à être recueilli en totalité en vue d'un dosage et l'autre étant destiné à être évacué,
caractérisé en ce que les moyens de pompage consistent en une seule pompe du type péristaltique et les moyens de répartition comprennent un sélecteur, ladite pompe étant reliée en entrée par une première canalisation à la sortie du moyen de recueil et de stockage et en sortie, par une deuxième canalisation, audit sélecteur, la première canalisation et la deuxième canalisation formant une canalisation unique traversant la pompe, et en ce que ledit moyen de recueil et de stockage, la première et la deuxième canalisation sont à usage unique

Dans des modes de réalisation avantageux, on a par ailleurs et/ou de plus recours à l'une et/ou à l'autre des dispositions suivantes :
- la canalisation reliant le moyen de recueil et de stockage à la sortie du dialyseur et la canalisation reliée à la sortie du sélecteur et par laquelle s'écoule le flux destiné à être recueilli, sont toutes deux à usage unique;
- la pompe et le sélecteur sont commandés par des moyens de commande communs;
- le moyen de recueil et de stockage consiste en une poche souple en matière plastique;
- le moyen de recueil et de stockage est muni d'un détecteur de niveau haut et d'un détecteur de niveau bas;
- le dispositif comporte des moyens de commande pour activer les moyens de pompage lorsque le liquide est au-dessus du détecteur de niveau haut et arrêter les moyens de pompage, lorsque le liquide est au-dessous du détecteur de niveau bas;
- le dispositif comporte des moyens de recueil du liquide échantillonné;
- le dispositif comporte des moyens de calcul du volume du liquide échantillonné et/ou du volume total du liquide usagé provenant du dialyseur.

L'invention sera mieux comprise à la lecture de la description qui sera faite au regard des dessins annexés dans lesquels :
La figure 1 est une vue schématique illustrant le principe de fonctionnement du dispositif selon l'invention.
La figure 2 est une vue schématique illustrant un exemple de réalisation du dispositif selon l'invention.

Les éléments communs aux figures seront désignés par les mêmes références.

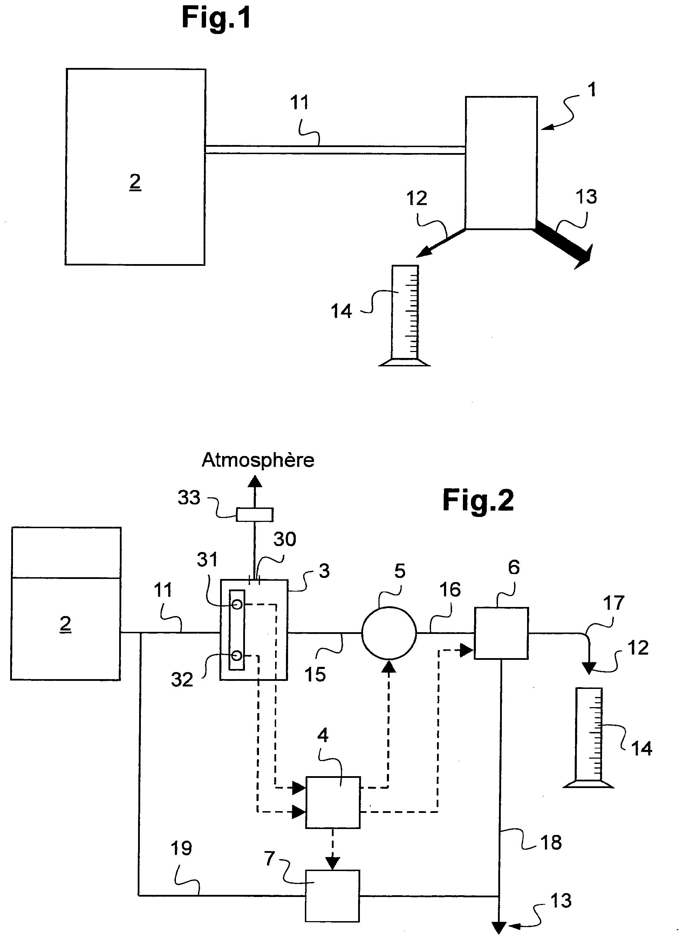

En référence tout d'abord à la figure 1, le dispositif 1 selon l'invention est connecté, en entrée, à la sortie d'un équipement de dialyse ou rein artificiel 2 par l'intermédiaire d'une canalisation 11. Ainsi, le dispositif 1 reçoit la totalité du liquide de dialyse usé en provenance du rein artificiel 2 et sépare ce liquide en deux flux 12 et 13 qui ont la même composition.

Le premier flux 12 est destiné à être recueilli en totalité, dans un récipient approprié 14, en vue d'un dosage. Ce récipient 14 peut consister en récipient ouvert ou de préférence, en une poche souple.

Le deuxième flux 13 est destiné à être rejeté directement à l'égout.

Ainsi, la fonction du dispositif 1 est de créer ces deux flux, dans un rapport de débit approprié, généralement de l'ordre de 100.

La figure 2 représente un exemple de réalisation du dispositif selon l'invention.

Le dispositif 1 comporte tout d'abord un récipient 3 de recueil et de stockage du liquide de dialyse usagé. Il reçoit ce liquide du dialyseur 2, par l'intermédiaire de la canalisation 11.

A sa partie supérieure, le récipient 3 comporte une ouverture 30 qui est reliée à l'atmosphère, directement ou grâce à une canalisation. Cette ouverture 30 permet l'évacuation des bulles éventuellement présentes dans le liquide recueilli dans le récipient 3.

Le récipient 3 comporte également un détecteur de niveau haut 31 et un détecteur de niveau bas 32. Chacun de ces détecteurs sont susceptibles d'envoyer un signal à l'unité de commande 4, lorsque le liquide atteint le niveau correspondant au détecteur en cause.

Il peut notamment s'agir de capteurs inductifs.

Ce récipient 3 reçoit donc la totalité du liquide de dialyse qui s'écoule en sortie du rein artificiel 2.

Il constitue un réservoir tampon destiné à contenir en permanence un volume de liquide, dont la valeur maximale correspond au niveau haut détecté par le détecteur 31.

La sortie du récipient 3 est reliée, par une canalisation 15, à des moyens de pompage 5.

La sortie de ces moyens de pompage est, quant à elle, reliée au moyen d'une canalisation 16 à un séparateur 6 dont la fonction est de répartir le liquide pompé par la pompe 5 en deux flux s'écoulant en sortie du sélecteur 6, le flux 12 par la canalisation 17 et le flux 13 par la canalisation 18.

Le récipient 3 est à usage unique. Il peut notamment s'agir d'une poche en matière plastique qui est positionnée en regard de chacun des détecteurs 31, 32.

Les canalisations 11, 15, 16 et 17 sont également, de préférence, des canalisations à usage unique. Elles sont notamment réalisées en silicone.

Ceci permet de s'affranchir des opérations de nettoyage et/ou désinfection qui sont nécessaires avec les dispositifs connus.

En effet, le liquide de dialyse usagé conduit à la formation d'un biofilm dans un délai rapide (environ 36 heures), ce qui entraine une contamination à la fois du récipient et des canalisations. Ceci est susceptible de perturber les mesures et de fausser la détection des substances d'intérêt dans l'échantillon.

Or, ces opérations de nettoyage et/ou désinfection sont longues et ne sont pas toujours efficaces.

De plus, lorsque les canalisations sont en matière plastique, on peut constater des détériorations, notamment des fissurations ou des déformations, comme des modifications de leur section, ce qui peut conduire à des erreurs lors de la détermination du volume de liquide pour chacun des flux 12 et 13.

Ces inconvénients sont également évités avec l'utilisation de canalisations à usage unique.

Par ailleurs, les moyens de pompage 5 sont constitués par une pompe péristaltique, entrainée par un moteur pas à pas (non illustré sur la figure 2).

De manière connue, une pompe péristaltique comporte une tête qui soutient un tuyau flexible et un rotor avec des galets qui déforment et obturent le tuyau pendant la rotation, de façon à déplacer le fluide se trouvant entre deux galets.

En pratique, avec une telle pompe, les canalisations 15 et 16 forment une canalisation unique traversant la pompe 5.

L'unité de commande 4 commande le moteur pas à pas de la pompe 5 grâce à des impulsions.

Un intérêt de cette pompe péristaltique est d'éviter tout contact entre le liquide transitant par la canalisation et la pompe elle-même. Ainsi, les risques de contamination du fluide sont limités et la pompe ne nécessite pas d'opération de nettoyage.

L'utilisation de canalisations à usage unique présente un intérêt particulier avec ce type de pompe car son fonctionnement peut, à terme, entrainer une usure mécanique des canalisations.

En effet, les galets écrasent la canalisation qui traverse la pompe, ce qui entraine une modification de la section de la canalisation.

Or, la quantité de liquide présente entre deux galets est fonction de la section de la canalisation. Ainsi, des mesures précises ne peuvent pas être assurées dans le temps.

Le fait de prévoir des canalisations à usage unique, notamment les canalisations 15 et 16, permet de résoudre ce problème.

C'est pourquoi l'utilisation de canalisations à usage unique, en combinaison avec une pompe péristaltique rend le dispositif selon l'invention d'autant plus efficace, fiable et précis.

Le séparateur 6 peut notamment consister en une canalisation à deux voies raccordée en amont à la canalisation 16 et associée à une électrovanne, notamment une électrovanne à pincement de voies qui permet de répartir le liquide dans les deux canalisations 17 et 18.

Le séparateur 6 est également commandé par l'unité de commande 4, en fonction de la commande de la pompe 5.

De façon plus précise, le dispositif fonctionne de la façon suivante.

Le dialyseur 2 étant en fonctionnement, le récipient 3 reçoit le liquide de dialyse usagé qui s'écoule depuis le dialyseur 2 par la canalisation 11.

Ce liquide s'accumule dans le récipient 3, en fonction du débit du dialyseur.

Lorsque le niveau du liquide dans le récipient 3 passe au-dessus du niveau haut, détecté par le détecteur 31, ce dernier envoie un signal à l'unité de commande 4, laquelle envoie des impulsions au moteur pas à pas qui va pouvoir entrainer la pompe 5.

Cette dernière pompe ainsi, par la canalisation 15, le liquide présent dans le récipient 3 et le transmet au sélecteur 6, par la canalisation 16.

L'unité de commande 4 commande le sélecteur de façon à ce que celui-ci divise le flux de liquide en entrée en deux flux 17 et 18 dans un rapport de débit déterminé, généralement de 100.

Le sélecteur 6 est commandé en fonction de la commande du moteur de la pompe 5.

Ainsi, par exemple, une électrovanne à pincement sera commandée en fonction des impulsions transmises au moteur pas à pas d'une pompe péristaltique.

En pratique, chaque pas du moteur correspond à un volume de liquide usagé qui est connu. Grâce à une commande appropriée de l'électrovanne pendant 100 impulsions successives, le séparateur orientera le flux issu de la canalisation 16 dans la canalisation 18 qui débouche à l'égout et, la 101^{ème} impulsion, le flux sera orienté vers la canalisation 17. Une telle commande permet d'assurer un rapport de 100 entre les flux 13 et 12. D'autres rapports peuvent être choisis.

Cette commande commune des moyens de pompage 5 et du séparateur 6 permet d'assurer une grande précision dans la détermination du volume du liquide destiné à l'échantillon et celle passant directement à l'égout.

Les essais réalisés montrent que le volume total de liquide usé et le volume de l'échantillon peuvent être déterminés avec une précision d'environ 3 %.

Par ailleurs, le liquide recueilli dans le récipient 14 pour constituer l'échantillon est identique à celui qui est rejeté à l'égout. L'échantillon qui fera ensuite l'objet d'une analyse sera donc représentatif de l'ensemble du liquide provenant du dialyseur 2.

Lorsque le niveau de liquide passe au-dessous du niveau bas détecté par le détecteur 32, celui-ci émet un signal qui est transmis à l'unité de commande 2, laquelle commande l'arrêt de la pompe 5.

Dans le cas d'une pompe péristaltique, l'unité de commande 4 arrête le moteur pas à pas commandant cette pompe.

La pompe 5 sera de nouveau mise en marche lorsque le liquide, provenant du dialyseur 2 et recueilli dans le récipient 3, aura atteint le niveau haut détecté par le détecteur 31.

Le détecteur selon l'invention est donc indépendant du fonctionnement du dialyseur 2, grâce au récipient 3 qui forme un réservoir tampon.

Par ailleurs, en fonction du temps de remplissage du récipient 3, l'unité de commande 4 peut commander la pompe 5 pour faire varier son débit en sortie.

L'ouverture 30 peut également être munie d'un système de sécurité 33 fonctionnant en cas de débordement du récipient 3 dû à un débit excessif de liquide usé, compte tenu des caractéristiques de la pompe, ou à un mauvais fonctionnement de la pompe ou de son moteur.

Le dispositif selon l'invention comporte, de préférence, une canalisation supplémentaire 19 entre la canalisation 11 et la canalisation 18. Une électrovanne 7 pilotée par l'unité de commande 4 permet de conduire l'ensemble du liquide fourni par le dialyseur 2 dans la canalisation 18 et donc à l'égout, lorsque le dispositif n'est pas utilisé ou en cas de panne sévère de la pompe ou de son moteur.

Les signes de références insérées après les caractéristiques techniques figurant dans les revendications ont pour seul but de faciliter la compréhension de ces dernières et ne sauraient en limiter la portée.

## Revendications

1. Dispositif de recueil d'un échantillon représentatif du liquide de dialyse en sortie d'un dialyseur (2) comportant :
- un moyen (3) de recueil et de stockage du liquide de dialyse usagé, destiné à être relié par une canalisation (11) à la sortie du dialyseur (2),
- des moyens de pompage (5) pour pomper du liquide stocké dans le moyen (3) de recueil et de stockage,
- des moyens (6) de répartition du liquide en deux flux différents, l'un (12) destiné à être recueilli en totalité en vue d'un dosage et l'autre (13) étant destiné à être évacué,
**caractérisé en ce que** les moyens de pompage (5) consistent en une seule pompe du type péristaltique et les moyens de répartition comprennent un sélecteur (6), ladite pompe étant reliée en entrée par une première canalisation (15) à la sortie du moyen (3) de recueil et de stockage et en sortie, par une deuxième canalisation (16), audit sélecteur (6), la première canalisation (15) et la deuxième canalisation (16) formant une canalisation unique traversant la pompe, et **en ce que** ledit moyen (3) de recueil et de stockage, la première et la deuxième canalisation sont à usage unique.

2. Dispositif selon la revendication 1, **caractérisé en ce que** la canalisation (11) reliant le moyen (3) de recueil et de stockage à la sortie du dialyseur et la canalisation (17) reliée à la sortie du sélecteur (6) et par laquelle s'écoule le flux (12) destiné à être recueilli, sont toutes deux à usage unique.

3. Dispositif selon la revendication 1 ou 2, **caractérisé en ce que** la pompe (5) et le sélecteur (6) sont commandés par des moyens de commande (4) communs.

4. Dispositif selon l'une des revendications 1 à 3, **caractérisé en ce que** le moyen (3) de recueil et de stockage consiste en une poche souple en matière plastique.

5. Dispositif selon l'une des revendications 1 à 4, **caractérisé en ce que** le moyen (3) de recueil et de stockage est muni d'un détecteur de niveau haut (31) et d'un détecteur de niveau bas (32).

6. Dispositif selon l'une des revendications 1 à 5, **caractérisé en ce qu'**il comporte des moyens de commande (4) pour activer les moyens de pompage (5) lorsque le liquide est au-dessus du détecteur de niveau haut (31) et arrêter les moyens de pompage, lorsque le liquide est au-dessous du détecteur de niveau bas (32).

7. Dispositif selon l'une des revendications 1 à 6, **caractérisé en ce qu'**il comporte des moyens de recueil (14) du liquide échantillonné.

8. Dispositif selon l'une des revendications 1 à 7, **caractérisé en ce qu'**il comporte des moyens de calcul du volume du liquide échantillonné et/ou du volume total du liquide usagé provenant du dialyseur.

## Patentansprüche

1. Vorrichtung zur Probensammlung, die repräsentativ für die Dialyseflüssigkeit am Ausgang eines Dialysegeräts (2) ist, umfassend:
- ein Mittel (3) zur Sammlung und Speicherung der verwendeten Dialyseflüssigkeit, die ausgelegt ist, um durch einen Kanal (11) mit dem Ausgang des Dialysegeräts (2) verbunden zu sein,
- Pumpmittel (5), um gespeicherte Flüssigkeit in das Mittel (3) zur Sammlung und Speicherung zu pumpen,
- Mittel (6) zur Verteilung der Flüssigkeit in zwei verschiedene Ströme, einen (12), der ausgelegt ist, um in seiner Gesamtheit im Hinblick auf eine Dosierung gesammelt zu werden, und wobei der andere (13) ausgelegt ist, um abgeleitet zu werden,
**dadurch gekennzeichnet, dass** die Pumpmittel (5) aus einer einzigen Pumpe vom Typ peristaltische Pumpe bestehen, und die Verteilungsmittel einen Selektor (6) umfassen, wobei die Pumpe am Eingang durch einen ersten Kanal (15) mit dem Ausgang des Mittels (3) zur Sammlung und Lagerung verbunden und am Ausgang durch einen zweiten Kanal (16) mit dem Selektor (6) verbunden ist, wobei der erste Kanal (15) und der zweite Kanal (16) einen einzigen Kanal bilden, der die Pumpe quert, und dadurch, dass das Mittel (3) zur Sammlung und Speicherung, der erste und der zweite Kanal zu einmaligen Verwendung sind.

2. Vorrichtung nach Anspruch 1, **dadurch gekennzeichnet, dass** der Kanal (11), der das Mittel (3) zur Sammlung und Speicherung mit dem Ausgang des Dialysegeräts verbindet, und der Kanal (17), der mit dem Ausgang des Selektors (6) verbunden ist, und durch den der Strom (12) abläuft, der ausgelegt ist, um gesammelt zu werden, beide zur einmaligen Verwendung sind.

3. Vorrichtung nach Anspruch 1 oder 2, **dadurch gekennzeichnet, dass** die Pumpe (5) und der Selektor (6) durch gemeinsame Steuermittel (4) gesteuert werden.

4. Vorrichtung nach einem der Ansprüche 1 bis 3, **dadurch gekennzeichnet, dass** das Mittel (3) zur Sammlung und Speicherung aus einer elastischen Tasche aus Plastikmaterial besteht.

5. Vorrichtung nach einem der Ansprüche 1 bis 4, **dadurch gekennzeichnet, dass** das Mittel (3) zur Sammlung und Speicherung mit einem Detektor von hohem Niveau (31) und einem Detektor von niedrigem Niveau (32) ausgestattet ist.

6. Vorrichtung nach einem der Ansprüche 1 bis 5, **dadurch gekennzeichnet, dass** sie Mittel zur Steuerung (4) umfasst, um die Pumpmittel (5) zu aktivieren, wenn sich die Flüssigkeit oberhalb des Detektors von hohem Niveaus (31) befindet, und die Pumpmittel zu stoppen, wenn sich die Flüssigkeit unterhalb des Detektors von niedrigem Niveau (32) befindet.

7. Vorrichtung nach einem der Ansprüche 1 bis 6, **dadurch gekennzeichnet, dass** sie Mittel zur Sammlung (14) der als Probe entnommenen Flüssigkeit umfasst.

8. Vorrichtung nach einem der Ansprüche 1 bis 7, **dadurch gekennzeichnet, dass** sie Mittel zur Berechnung des Volumens der als Probe entnommenen Flüssigkeit und/oder des Gesamtvolumens der verwendeten Flüssigkeit, die aus dem Dialysegerät stammt, umfasst.

## Claims

1. Device for collecting a representative sample of dialysis fluid at the output of a dialyser (2) including:
- means (3) for collecting and storing the waste dialysis fluid, intended to be connected by a conduit (11) to the output of the dialyser (2),
- pumping means (5) for pumping fluid stored in the collection and storage means (3),
- means (6) for splitting the fluid into two different flows, one (12) intended to be collected in full with a view to proportioning and the other (13) being intended to be discharged,
**characterised in that** the pumping means (5) consist of a single pump of the peristaltic type and the splitting means comprise a selector (6), said pump being connected at the input by a first conduit (15) to the output of the collection and storage means (3) and at the output, by a second conduit (16), to said selector (6), the first conduit (15) and the second conduit (16) forming a single conduit traversing the pump, and **in that** said collection and storage means (3), the first and the second conduit are single-use.

2. Device according to claim 1, **characterised in that** the conduit (11) connecting the collection and storage means (3) to the output of the dialyser and the conduit (17) connected to the output of the selector (6) and whereby the flow (12) intended to be collected flows, are both single-use.

3. Device according to claim 1 or 2, **characterised in that** the pump (5) and the selector (6) are controlled by common control means (4).

4. Device according to one of claims 1 to 3, **characterised in that** the collection and storage means (3) consists of flexible plastic pouch.

5. Device according to one of claims 1 to 4, **characterised in that** the collection and storage means (3) is equipped with a high-level detector (31) and a low-level detector (32).

6. Device according to one of claims 1 to 5, **characterised in that** it includes control means (4) for activating the pumping means (5) when the fluid is above the high level detector (31) and shutting down the pumping means, when the fluid is below the low level detector (32).

7. Device according to one of claims 1 to 6, **characterised in that** it includes means (14) for collecting the sampled fluid.

8. Device according to one of claims 1 to 7, **characterised in that** it includes means for computing the volume of the sampled fluid and/or the total volume of waste fluid from the dialyser.
